# EUROPEAN PATENT SPECIFICATION

(11) **EP 1 528 920 B1**
(45) Date of publication and mention of the grant of the patent: **24.07.2013**
(21) Application number: 02758830.0
(22) Date of filing: 12.08.2002
(51) Int. Cl.: A61K 31/198, A61P 33/00

(54) **Suppression of abnormal proliferation of prion proteins with leucine, isoleucine or valine**
Hemmung der abnormalen Prionproteinsproliferation mit Leucine, Isoleucine oder Valine
Inhibition de la proliferation anormale des protéines prioniques, faisant intervenir de la leucine, de l'isoleucine ou de la valine

(43) Date of publication of application: 11.05.2005
(73) Proprietor: Murayama, Yuuichi, Tsukuba-shi, Ibaraki 305-0035 (JP); Moriyama, Masami, Yokohama-shi, Kanagawa 232-0064 (JP)
(72) Inventor: Murayama, Yuuichi, Tsukuba-shi, Ibaraki 305-0035 (JP); Moriyama, Masami, Yokohama-shi, Kanagawa 232-0064 (JP)
(74) Representative: Albrecht, Thomas
(86) International application number: PCT/JP2002/008209
(87) International publication number: WO 2004/016261

(56) References cited:
- EP-A- 0 764 442
- EP-A- 0 824 919
- WO-A-00/64420
- WO-A-01/26623
- WO-A-96/21437
- US-A- 5 919 823
- CALLEBAUT, ISABELLE ET AL: "Common prevalence of alanine and glycine in mobile reactive center loops of serpins and viral fusion peptides: do prions possess a fusio peptide?" JOURNAL OF COMPUTER-AIDED MOLECULAR DESIGN (1994), 8(2), 175-91 , XP008015408
- DATABASE WPI Section Ch, Week 200264 Derwent Publications Ltd., London, GB; Class B05, AN 2002-599522 XP002236107 & WO 02 49638 A (AJINOMOTO KK), 27 June 2002 (2002-06-27)
- ANDERSEN, AVINS: "lOWERING BRAIN PHENYLALANINE LEVELS ...", ARCH. NEUROL., vol. 33, 1976,

## Description

### Technical Field

The present invention relates to suppressing proliferation of abnormal prion proteins, and more particularly, to suppressing proliferation of abnormal prion proteins through administration of essential amino acids having a branched side chain.

### Background Art

Scrapie, the first to be discovered of a group of diseases caused by proliferation of abnormal prion protein, is found in sheep and impairs motor functions of the animal. Scrapie is characterized by porous, sponge-like appearance of affected brains. Other diseases similarly resulting from proliferation of abnormal prion protein were later identified, including bovine spongiform encephalopathy (BSE) in cows, commonly known as mad cow disease, Creutzfeldt-Jakob disease (CJD), and Gerstmann-Straussler-Scheinker syndrome (GSS) in humans.

These diseases were not caused by viral infection, nor had any known pathogen been identified to cause them. However, a specific type of protein commonly found in individuals suffering the disease was suspected to be a pathogenic agent involved in the transmission, or infection, of the disease. The protein was hypothetically named as a prion, or small proteinaceous infectious particle, and the group of diseases caused by the agent has come to be collectively referred to as prion diseases.

Of the known prion diseases, GSS is known to be a familial disease and has proven to be triggered at 100% probability by a point mutation in a prion protein in which one amino acid of the protein is substituted for another type of amino acid (leucine).

The gene encoding prion protein, which consists of 235 amino acids, is located on chromosome 20. The infectious factor of the prion diseases is thought to mostly consist of this prion protein. The type of prion protein that acts as the infectious factor is termed as scrapie-type prion or abnormal prion (PrP^{SC}) while the normal prion is referred to as PrP.

As with the case of GSS, a point mutation in the prion protein can cause onset of other diseases. For example, substitution of proline (Pro) for leucine (Leu) in codon 102 of prion protein triggers cerebellar ataxia; substitution of proline (Pro) for leucine (Leu) in codon 102/219 (Lys) causes dementia or cerebellar ataxia, and substitution of proline (Pro) for leucine (Leu) in codon 105/129 (Val) causes spastic tetraplegia.

Also, in patients suffering fatal familial insomnia (FFI), a prion disease characterized by persistent insomnia and deaths within one year, aspartic acid (Asp) has been substituted for asparagine (Asn) in codon 178 of the prion protein.

Similar diseases caused by mutant prion proteins are also found in various animal species and are observed to transmit from one animal species to another via abnormal prion protein. For example, animal feed contaminated with materials prepared from scrapie-infected sheep has given rise to BSE in cows. Similarly, cat food containing materials prepared from BSE-infected cows has caused feline spongiform encephalopathy (FSE) in cats. Recent studies suggest that there is a significant chance that prion diseases, particularly BSE, are transmitted to human (Collinge J. et. al., Nature 383: 685-690, 1996).

While the manner by which the prion diseases are transmitted via abnormal prion proteins has yet to be clearly understood, it is known that an abnormal prion, despite its nature as protein, retains its infectivity after undergoing sterilizing processes under high pressure or by drying. Furthermore, abnormal prions are not denatured through treatment with formalin, alcohol or phenol. For these reasons, when a person is infected with abnormal prion protein, it is necessary to minimize proliferation of the abnormal prion in order to prevent onset of the disease. However, no effective approach has ever been developed that can suppress the proliferation of abnormal prion protein, nor has any attempt been made to this date to prevent onset of the disease based on such approaches.

### Disclosure of Invention

In view of the present state of the art, it is a primary objective of the present invention to suppress proliferation of abnormal prion protein in animals, including humans, infected with the prion. Also, it is an ultimate goal of the present invention to prevent onset of prion diseases in animals, including humans, infected with the prion.

In an effort to find a way to achieve these objectives, the present inventors have made a finding that proliferation of abnormal prion protein is effectively suppressed by essential amino acids, in particular those having a branched side chain, and thus investigated the possibility of these essential amino acids as a viable suppressive agent for suppressing proliferation of abnormal prion protein. The finding ultimately led the present inventors to bring the present invention to completion.

Accordingly, one essential aspect of the present invention is suppressing proliferation of abnormal prion proteins in the prevention or treatment of a prion disease. This involves systemically, orally, intracerebrally or intraspinally administering an essential amino acid having a branched side chain at a high concentration.

Specifically, examples of the essential amino acid having a branched side chain for use in the present invention include leucine (Leu), isoleucine (Ile), and valine (Val) and a mixture thereof. The present inventors discovered that, of these amino acids, leucine is particularly effective in suppressing proliferation of abnormal prion protein.

Accordingly, a most preferred embodiment of the present invention is suppressing proliferation of abnormal prion proteins, which involves systemically, orally, intracerebrally or intraspinally administering leucine at a high concentration. in the prevention or treatment of a prion disease.

A second essential aspect of the present invention is administering an essential amino acid, involving systemically, orally, intracerebrally or intraspinally administering an essential amino acid having a branched side chain at a high concentration so as to suppress proliferation of abnormal prion proteins; in the prevention or treatment of a prion disease

More specifically, the essential amino acid having a branched side chain to be administered systemically, orally, intracerebrally or intraspinally for the purpose of suppressing proliferation of abnormal prion protein is selected from the group consisting of leucine, isoleucine, valine and a mixture thereof, and most preferably, leucine.

A third essential aspect of the present invention is a suppressive agent for suppressing proliferation of abnormal prion proteins in the prevention or treatment of a prion disease. The suppressive agent contains as an active ingredient an essential amino acid having a branched side chain.

In one specific embodiment, the suppressive agent contains, as the essential amino acid having a branched side chain to serve as an active ingredient, one selected from the group consisting of leucine, isoleucine, valine, and a mixture thereof.

In the most preferred embodiment, the suppressive agent contains leucine as an active ingredient.

A fourth essential aspect of the present invention is a use of an essential amino acid having a branched side chain for being administered systemically, orally, intracerebrally or intraspinally so as to suppress proliferation of abnormal prion proteins, in the prevention or treatment of a prion disease.

Specifically, the fourth aspect of the present invention concerns the use of leucine, isoleucine, valine, or a mixture thereof, and most preferably, the use of leucine as the essential amino acid having a branched side chain to be administered systemically, orally, intracerebrally or intraspinally so as to prevent proliferation of abnormal prion protein, in the prevention or treatment of a prion disease.

### Brief Description of Drawing

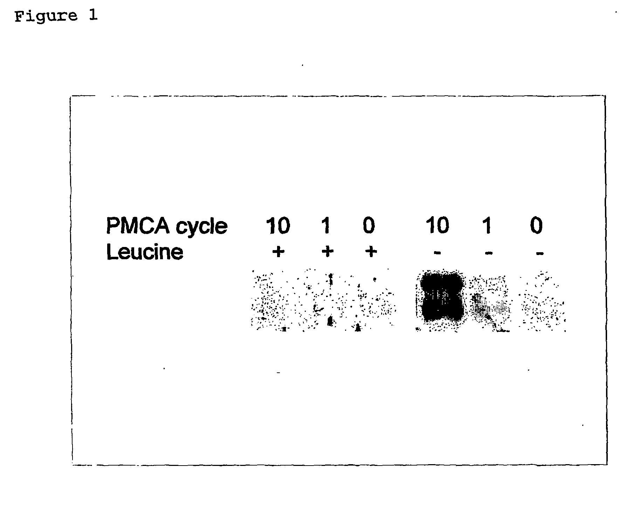

Figure 1 is a photograph showing the results of luminescence reactions obtained in Test Example 1 using ECL Western blotting technique.

### Best Mode Carrying Out the Invention

One essential aspect of the present invention is preventing proliferation of an abnormal prion protein through systemic, oral, intracerebral, or intraspinal administration of an essential amino acid having a branched side chain, specifically, one selected from the group consisting of leucine, isoleucine, valine, and a mixture thereof at a high concentration.

The essential amino acid having a branched side chain, or specifically, one selected from the group consisting of leucine, isoleucine and valine, is a safe compound, and the fact that essential amino acids promote growth of human body and help us stay healthy makes the use of the present invention highly effective.

Of the different essential amino acids with a branched side chain, use of leucine has resulted in particularly favorable results. Considering the fact that the infectious, abnormal prion proteins result from a point mutation occurring in the normal prion protein, in particular, substitution of one amino acid for leucine, it is thought that administering leucine can somehow help prevent proliferation of abnormal prion protein.

Thus, in order to suppress proliferation of abnormal prion protein, the essential amino acid having a branched side chain, such as leucine, may be systemically administered through injection. Alternatively, it may be administered orally, intracerebrally or intraspinally. In the latter two cases, the essential amino acid is delivered to brain tissue or spinal cord.

The dosage of the essential amino acid is not limited to a particular range and can be any amount sufficient to suppress proliferation of abnormal prion protein. For example, it may be administered at a large dose of 5 to 15g/kg. The essential amino acid may be administered as a solution having a high concentration of 20 to 40mg/mL or, in the case of oral administration, as a solid preparation. In general, the essential amino acid can be administered in the form of a solution with a proper concentration, tablet, powder preparation or other suitable preparations.

### Examples

The present invention will now be described in detail with reference to the results of a test in which an abnormal prion protein derived from scrapie-infected sheep was used and suppression of proliferation of the protein was observed.

### Test Example 1:

Effects that the essential amino acids having a branched side chain have on the proliferation reaction of abnormal prion protein were examined in the following experiment.

### 1. Method:

Using the protein misfolding cyclic amplification technique (PMCA), abnormal prion protein was amplified *in vitro.* Brains of sheep collected from a healthy sheep and a scrapie-infected sheep were independently homogenized in 0.5% Triton X-0.05% SDS-PBS (containing a protease inhibitor) to form 10%(w/v) solutions. To each of 10µL aliquots of the brain homogenate derived from healthy sheep, one-hundredth as much of the brain homogenate derived from scrapie-infected sheep was added to perform PMCA amplification. Leucine was added to a group of the resulting mixtures to a final concentration of 15mg/mL.

Each mixture of the brain homogenates was incubated for 1 hour while being stirred at 37°C and was then sonicated using Digital Sonifier 450D available from Branson. Sonication was performed by setting power output to 100% and repeating 5 times a cycle of 0.2-second oscillation period followed by 0.1-second interval.

The mixtures with and without leucine were individually subjected to 0 time (control), 1 time and 10 times of the incubation-sonication treatment to give samples. Proteinase K (PK) was added to each sample to a final concentration of 50ug/mL, and the samples were incubated for 1 hour at 37°C.

Abnormal prion protein was detected by Western blotting technique. Each sample was separated and electrophoresed by 10% SDS-PAGE and then transferred to a membrane. After blocking nonspecific binding sites on the membrane, each membrane was incubated with rabbit anti-PrP₉₄₋₁₁₂ antibody for 1 hour at room temperature, washed, and then incubated with HRP-labeled anti-rabbit IgG antibody. The membranes were then washed and luminescence was detected by ECL + plus.

### 2. Results

After PMCA, bands of PK resistant fragments were significantly enhanced in the leucine-free group (e.g., 5-fold in the sample subjected to 10 cycles of the treatment). The degree of enhancement was dependent on the number of cycles.

In contrast, no prominent bands were formed suggesting the presence of PK resistant fragments in the leucine-treated group, including the sample subjected to 10 cycles of PMCA.

Collectively, the results suggest that addition of leucine has effectively suppressed proliferation of abnormal prion protein *in vitro.*

Shown in the figure 1 are the results of luminescence reaction performed by ECL + plus. The results obtained for the leucine-treated group are shown on the left of the figure and the results for the leucine-free group on the right.

### Industrial Applicability

As set forth, the use of the present invention to administer essential amino acids having a branched side chain, in particular leucine, can suppress proliferation of abnormal prion proteins.

In view of the fact that no effective measure has been proposed to this date to suppress proliferation of abnormal prion proteins, the suppression of proliferation of abnormal prions, made possible by the present invention, will ultimately lead to prevention of the onset of prion diseases in animals, including humans, infected with an abnormal prion protein and thus proposes a possibility for the treatment of various prion diseases now attracting significant public attention, such as BSE in cows and CJD in humans.

## Claims

1. Use of an essential amino acid having a branched side chain as an active ingredient for the manufacture of a medicament for suppressing proliferation of abnormal prion proteins in the prevention or treatment of a prion disease, wherein said prion disease is selected from the group consisting of scrapie, bovine spongiforme encephalopathy (BSE), and Gerstmann-Straussler-Scheinker syndrome.

2. The use according to claim 1, wherein the essential amino acid is selected from the group consisting of leucine, isoleucine, valine, and a mixture thereof.

3. The use according to claim 1, wherein the essential amino acid having a branched side chain is leucine.

4. The use according to any one of the claims 1 to 3, where said medicament is formulated for systemical, oral, intracerebral or intraspinal administration.

5. Use of an essential amino acid having a branched side chain as an active ingredient for the manufacture of a medicament for suppressing proliferation of abnormal prion proteins in the prevention or treatment of Creutzfeldt-Jakob disease, wherein said medicament is formulated for systemical, intracerebral or intraspinal administration, and wherein the essential amino acid is selected from the group consisting of leucine, isoleucine, valine, and a mixture thereof.

6. The use according to claim 5, wherein the essential amino acid having a branched side chain is leucine.

## Patentansprüche

1. Verwendung einer essentiellen Aminosäure, die eine verzweigte Seitenkette hat, als aktives. Ingrediens für die Herstellung eines Medikaments zum Supprimieren der Proliferation von abnormalen Prionproteinen bei der Prävention oder Behandlung einer Prionenkrankheit, wobei die Prionenkrankheit aus der Gruppe, bestehend aus Traberkrankheit, boviner spongiformer Enzephalopathie (BSE) und Gerstmann-Sträussler-Scheinker-Syndrom, ausgewählt ist.

2. Verwendung gemäß Anspruch 1, wobei die essentielle Aminosäure aus der Gruppe, bestehend aus Leucin, Isoleucin, Valin und einem Gemisch davon, ausgewählt ist.

3. Verwendung gemäß Anspruch 1, wobei die essentielle Aminosäure, die eine verzweigte Seitenkette hat, Leucin ist.

4. Verwendung gemäß einem der Ansprüche 1 bis 3, wobei das Medikament zur systemischen, oralen, intrazerebralen oder intraspinalen Verabreichung formuliert ist.

5. Verwendung einer essentiellen Aminosäure, die eine verzweigte Seitenkette hat, als aktives Ingrediens für die Herstellung eines Medikaments zum Supprimieren der Proliferation von abnormalen Prionproteinen bei der Prävention oder Behandlung von Creutzfeldt-Jakob-Krankheit, wobei das Medikament zur systemischen, intrazerebralen oder intraspinalen Verabreichung formuliert ist und wobei die essentielle Aminosäure aus der Gruppe, bestehend aus Leucin, Isoleucin, Valin und einem Gemisch davon, ausgewählt ist.

6. Verwendung gemäß Anspruch 5, wobei die essentielle Aminosäure, die eine verzweigte Seitenkette hat, Leucin ist.

## Revendications

1. Utilisation d'un acide aminé essentiel ayant une chaîne latérale ramifiée en tant que principe actif pour la fabrication d'un médicament destiné à supprimer la prolifération de protéines prions anormales dans la prévention ou le traitement d'une maladie à prions, dans laquelle ladite maladie à prions est sélectionnée dans le groupe consistant en la tremblante du mouton, l'encéphalopathie spongiforme bovine (BSE), et le syndrome de Gerstmann-Stràussler-Scheinker.

2. Utilisation selon la revendication 1, dans laquelle l'acide aminé essentiel est sélectionné dans le groupe consistant en la leucine, l'isoleucine, la valine et un mélange de celles-ci.

3. Utilisation selon la revendication 1, dans laquelle l'acide aminé essentiel ayant une chaîne latérale ramifiée est la leucine.

4. Utilisation selon l'une quelconque des revendications 1 à 3, dans laquelle ledit médicament est formulé pour l'administration systémique, orale, intracérébrale ou intrarachidienne.

5. Utilisation d'un acide aminé essentiel ayant une chaîne latérale ramifiée en tant que principe actif pour la fabrication d'un médicament destiné à supprimer la prolifération de protéines prions anormales dans la prévention ou le traitement de la maladie de Creutzfeldt-Jakob, dans laquelle ledit médicament est formulé pour l'administration systémique, intracérébrale ou intrarachidienne, et dans laquelle l'acide aminé essentiel est sélectionné dans le groupe consistant en la leucine, de l'isoleucine, de la valine et d'un mélange de celles-ci.

6. Utilisation selon la revendication 5, dans laquelle l'acide aminé essentiel ayant une chaîne latérale ramifiée est la leucine.
